# EUROPÄISCHE PATENTSCHRIFT

(11) **EP 4 051 092 B1**
(45) Veröffentlichungstag und Bekanntmachung des Hinweises auf die Patenterteilung: **05.06.2024**
(21) Anmeldenummer: 20808294.1
(22) Anmeldetag: 29.10.2020
(51) Int. Cl.: A61B 5/00, A61N 5/06

(54) **LICHTAPPLIKATOR**
LIGHT APPLICATOR
APPLICATEUR DE LUMIÈRE

(30) Priorität: 01.11.2019 DE 102019129556
(43) Veröffentlichungstag der Anmeldung: 07.09.2022
(73) Patentinhaber: Richard Wolf GmbH, 75438 Knittlingen (DE)
(72) Erfinder: WAGNER, Stefan, 75015 Bretten (DE); HÄHNLE, Friedrich, 75015 Bretten (DE); LANGEN, Sebastian, 75438 Knittlingen (DE); LAMBERTZ, Matthias, 75015 Bretten (DE); WEBER, Bernd Claus, 76228 Karlsruhe (DE)
(74) Vertreter: Patentanwälte Hemmer Lindfeld Frese Partnerschaft mbB
(86) Internationale Anmeldenummer: PCT/DE2020/200095
(87) Internationale Veröffentlichungsnummer: WO 2021/083465

(56) Entgegenhaltungen:
- EP-A1- 2 449 994
- WO-A2-2014/068495
- CN-B- 109 331 346
- US-A- 5 445 608

## Beschreibung

Die vorliegende Offenbarung betrifft einen Lichtapplikator zur Untersuchung und/oder Behandlung eines organischen Körpers, insbesondere zur photodynamischen Therapie (PDT) von pathologischem Gewebe.

Es ist bekannt, Endoskope dazu zu nutzen, um Videoaufnahmen vom Inneren eines menschlichen oder tierischen Körpers zu Zwecken der medizinischen Diagnose und/oder Therapie zu machen. Dabei ist es ein ständiges Bestreben, den Einführabschnitt von Endoskopen möglichst dünn auszugestalten, damit möglichst kleine Hohlräume eingesehen werden können und das Gewebe dabei möglichst wenig in Mitleidenschaft zu ziehen.

Endoskope werden allerdings nicht nur dazu genutzt, um Bild-oder Videoaufnahmen zu machen, sondern auch als Diagnose- oder Therapiemittel selbst eingesetzt. Beispielsweise für die Detektion und Lokalisierung von prä- und frühmalignem Gewebe kann eine Fluoreszenz-Endoskopie eingesetzt werden, bei der es nicht auf eine natürliche Echtfarb-Darstellung des Gewebes ankommt, sondern lediglich auf eine Fluoreszenzanregung, mit der sich pathologisches Gewebe von gesundem Gewebe unterscheiden lässt. Dabei kann das mittels Lichtstrahlung angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise im Rahmen einer photodynamischen Diagnose (PDD) und/oder photodynamischen Therapie (PDT) mittels eines Photosensibilisators bzw. Markerstoffs durchgeführt werden, der sich selektiv an pathologischem Gewebe anreichert.

Bei der photodynamischen Therapie (PDT) wird Licht mittels eines Lichtapplikators unmittelbar auf oder sogar in pathologisches Gewebe appliziert, um lichtinduziert die Bildung von Sauerstoffradikalen mittels des örtlich begrenzt angereicherten Photosensibilisators bzw. Markerstoffs zu fördern und dadurch das pathologische Gewebe, wie etwa einen Tumor, zu zerstören. Typischerweise wird dazu Laserlicht in einen Lichtleiter eingekoppelt und zum Gewebe geleitet. Wenn das pathologische Gewebe flächig auf einer äußeren Oberfläche, z. B. der Haut, oder einer inneren Oberfläche, z. B. der Speiseröhreninnenfläche oder Darmwandung, angeordnet ist, dann kann das Therapielicht relativ einfach ausgekoppelt und auf die pathologische Gewebefläche gestrahlt werden. Erstreckt sich allerdings das pathologische Gewebe über ein Volumen, so kann wegen der begrenzten Eindringtiefe des Lichts in das Gewebe nicht immer ein Tumor von "außen" effektiv bestrahlt werden. In diesem Fall ist die PDT besonders effektiv, wenn das Licht vom Inneren des pathologischen Gewebevolumens aus möglichst isotrop abgestrahlt wird. Dazu muss der Lichtapplikator in das pathologische Gewebe eingestochen werden. Dies wird auch als interstitielle (durch innere Oberflächen) und/oder perkutane (durch die Haut) PDT bezeichnet.

In der EP 2 449 994 A1 ist beispielsweise beschrieben, wie ein Lichtapplikator in Form eines Laserlichtleiters in pathologisches Gewebe eingeschoben wird, nachdem der Weg für den Lichtleiter durch das Gewebe mit einer Nadel vorgestochen wurde. Die WO 2014/068495 A1 beschreibt einen Katheder mit lichtstreuendem Lichtleiter zur lateralen Abstrahlung und einer Hülse zum Verdecken der lateralen Abstrahlung. Die US 5,445,608 A beschreibt einen starren PDT-Lichtapplikator.

Nachteilig an den bekannten Lösungen ist, dass sich das zu behandelnde Organ während der PDD oder PDT gegenüber der Einstichstelle an der Haut bewegen kann. Zum Beispiel kann sich die Lunge durch die Atmung des Patienten relativ zur Einstichstelle stark bewegen. Zum einen verbleibt das distale Ende des Laserlichtleiters durch die Organbewegung während der PDD oder PDT nicht an der gewünschten Gewebestelle. Zum anderen kann gesundes Gewebe, das zuvor durchstochen werden musste, durch die Organbewegung mehr als nötig geschädigt werden. Außerdem ist die aus der EP 2 449 994 A1 bekannte Lösung relativ komplex und teuer, sodass sie nicht als Einweg-Artikel zum einmaligen Gebrauch realisierbar ist.

Daraus ergibt sich die Aufgabe, einen kostengünstigeren Lichtapplikator bereitzustellen, der eine effektive Lichtbestrahlung für eine möglichst gewebeschonende interstitielle und/oder perkutane PDD oder PDT von Gewebe in sich bewegenden Organen erlaubt.

Gemäß der vorliegenden Offenbarung wird zur Lösung dieses Problems ein Lichtapplikator zur Untersuchung und/oder Behandlung von einem organischen Körper bereitgestellt, wobei der Lichtapplikator einen distalseitigen Einführabschnitt mit einer LED am distalen Ende zum Einstechen in Gewebe des organischen Körpers aufweist, wobei der Einführabschnitt ein flexibles Leiterelement zur Stromversorgung der LED und eine das Leiterelement zumindest teilweise umgebende starre Einführhülse aufweist, wobei das Leiterelement relativ zur starren Einführhülse axial beweglich ist, wobei am distalseitigen Ende des Leiterelements ein radialer Vorsprung mit einem axialen Anschlag für ein distales Ende der Einführhülse angeordnet ist. Der radiale Vorsprung wird vorzugsweise durch eine starre Applikatorspitze gebildet, die mit dem distalseitigen Ende des Leiterelements verbunden ist.

Bei dem hier offenbarten Lichtapplikator wird also kein Laserlichtleiter eingesetzt, sondern das Diagnose- oder Therapielicht in situ am distalen Ende des Lichtapplikators durch eine miniaturisierte LED erzeugt, z.B. mit einer lateralen Breite von weniger als 1 mm. Ein teurer Laser wird daher nicht benötigt, sodass die Kosten stark reduziert sind. Der radiale Vorsprung am distalseitigen Ende des Leiterelements hat zum einen den Effekt, dass die Einführhülse beim Einstechen des Applikators am axialen Anschlag mit der starren Applikatorspitze anliegt und damit das distalseitige Ende des Leiterelements zusammen mit der LED mit Hilfe der Einführhülse genau an die gewünschte Behandlungsstelle positioniert werden kann. Die Einführhülse kann dann so weit wieder proximalwärts zurückgezogen werden, dass sie sich nicht mehr in dem sich bewegenden Organ befindet, aber noch als Kanalführung in der Haut verbleibt. Der radiale Vorsprung am distalseitigen Ende des Leiterelements hat dann den weiteren Effekt, dass das distalseitige Ende des Leiterelements mit der Applikatorspitze im Gewebe verankert bleibt und bei Organbewegungen mitgeführt wird. Das Leiterelement ist dazu möglichst flexibel ausgestaltet und möglichst reibungsarm für axiale Bewegungen in der Einführhülse geführt. Das distalseitige Ende des Leiterelements mit der Applikatorspitze und mit der LED verbleibt dadurch bei Organbewegungen an der gewünschten Behandlungsstelle, ohne dass dabei das gesunde Gewebe beschädigt wird.

Das flexible Leiterelement weist hier also keinen Lichtleiter für proximal eingekoppeltes Laserlicht auf, sondern kann hauptsächlich aus einem elektrischen Leiter in Form eines flexiblen Drahts und/oder Litzen und/oder eines flexiblen Koaxialkabels bestehen, wobei der elektrische Leiter eine distalseitig angeordnete LED mit Strom versorgt. Das flexible Leiterelement weist vorzugsweise außenseitig eine elektrische Isolierung in Form eines biokompatiblen Mantels und/oder einer biokompatiblen Beschichtung auf. Der radiale Vorsprung am distalen Ende des Leiterelements kann als Applikatorspitze zum gewebeschonenden Durchstechen der Haut und des proximal vor der Behandlungsstelle liegenden Gewebes dienen. Die Spitze zum gewebeschonenden Durchstechen der Haut ist also nicht Teil der Einführhülse, sondern am distalen Ende des Leiterelements angeordnet und wird mittels der am Anschlag anliegenden Einführhülse distalwärts geschoben. Dass die Spitze Teil des flexiblen Leiterelements und nicht Teil der Einführhülse ist, ist zudem von Vorteil, da somit die Gefahr einer Beschädigung des Leiterelements durch die Einführhülse reduziert ist. Der radiale Vorsprung kann als radiale Aufweitung, als Kante, als Steg oder anderweitig ausgebildet sein, um einen axialen Anschlag für ein distales Ende der Einführhülse zu bilden. Er kann umlaufend geschlossen, umlaufend segmentiert oder umlaufend punktuell ausgestaltet sein. Als radialer Vorsprung könnte beispielsweise ein radial vorstehender Pin genügen.

Der hierin offenbarte Lichtapplikator kann sehr günstig hergestellt werden und somit als steriler Einweg-Artikel zum einmaligen Gebrauch realisiert werden, was aufwendige Reinigungen und Sterilisationen beim Anwender obsolet macht. Bei größeren Tumoren oder ganzen pathologischen Organen oder Organbereichen kann eine Mehrzahl an hierin offenbarten Lichtapplikatoren gleichzeitig für die PDT eingesetzt werden, indem sie verteilt über das gesamte Organ eingestochen werden, um das gesamte Organ homogen auszuleuchten. Da sich der Photosensibilisator bzw. Markerstoff selektiv nur in pathologischem Gewebe anreichert und dort unter dem Lichteinfluss reagiert, wird gesundes Gewebe durch das Licht nicht beschädigt. Zum einen muss das pathologische Gewebe dann zuvor nicht mehr so genau lokalisiert werden und zum anderen wird das Risiko verringert, dass pathologisches Gewebe unbemerkt untherapiert bleibt. Für die perkutane PDT mit mehreren Lichtapplikatoren kann es sinnvoll sein, eine flächig vor oder auf die Haut des Patienten platzierbare und/oder klebbare organspezifische Schablone bzw. Template mit Markierungen und/oder Durchbrechungen bereitzustellen, um einem Anwender damit Einstichstellen, -winkel und/oder -tiefen für die Lichtapplikatoren anzuzeigen und eine möglichst vollständige und weitgehend homogene Ausleuchtung eines Organs zu erzielen.

Der Lichtapplikator kann allerdings nicht nur für die Therapie eingesetzt werden, sondern auch für die Untersuchung, also die Diagnose. Insbesondere im Zusammenspiel mit einem Endoskop oder mit dem Lichtapplikator als Teil eines Endoskops kann die durch den Lichtapplikator erzeugte Fluoreszenz eines in pathologischem Gewebe angereicherten Photosensibilisators bzw. Markerstoffs beobachtet werden.

Der Lichtapplikator kann durch einen Arbeitskanal eines endoskopischen Instruments geschoben werden und am distalen Ende mit seinem Einführabschnitt in das Gewebe gestochen werden, was vorzugsweise mit einem distalseitigen Bildsensor am endoskopischen Instrument beobachtbar ist. Dies ist insbesondere für die interstitielle PDT sinnvoll, wenn beispielsweise durch natürliche Körperöffnungen, wie etwa Darm, Harnleiter, Speise- oder Luftröhre der Weg zum Tumor mittels eines endoskopischen Instruments verkürzt werden kann. Der Lichtapplikator kann allerdings auch ganz ohne endoskopisches Instrument beispielsweise für die perkutane PDT verwendet werden, bei der der Einführabschnitt beispielsweise CT- oder ultraschallunterstützt von außen durch die Haut bis in das pathologische Gewebe gestochen wird. Der Einführabschnitt kann vorzugsweise eine oder mehrere abstrakte oder konkrete laterale Längenmarkierungen aufweisen, die dem Anwender beim Einstechen eine relative oder absolute Einstichtiefe vermitteln. Insbesondere kann der Applikator Längenmarkierungen für die relative Axialposition des Leiterelements und der Einführhülse zueinander aufweisen.

Optional kann der radiale Vorsprung zumindest teilweise radial über die Innenkontur der Einführhülse hinausragen, aber radial innerhalb der Außenkontur der Einführhülse liegen. Damit wird sowohl der durch das distale Ende der starren Einführhülse verursachte Widerstand beim Einstechen des Applikators als auch derdurch den Anschlag verursachte Widerstand beim Herausziehen des Applikators stark reduziert. Es wird also das zu durchstechende gesunde Gewebe weitestgehend geschont.

Optional kann der radiale Vorsprung ein oder mehrere Widerstandselemente zum Verankern des Vorsprungs im Gewebe aufweisen. Der Anschlag bildet vorzugsweise selbst ein Widerstandselement. Die Widerstandselemente können beispielweise als Widerhaken fungieren und einen höheren Widerstand bei axialer Bewegung in proximale Richtung verursachen als in distale Richtung.

Optional können die Widerstandselemente als Gewindestege ausgebildet sein. Vorzugsweise können die Gewindestege distalwärts verjüngend ausgebildet sein. Mit Gewindestegen als Widerstandselemente kann die Applikatorspitze in das maligne Gewebe eingeschraubt werden, damit die Applikatorspitze während der Therapie den Bewegungen des Organs folgt. Nach der Therapie kann die Applikatorspitze entsprechend wieder ausgeschraubt werden.

Optional kann der radiale Vorsprung einen in Umlaufrichtung wirksamen Formschluss mit dem distalen Ende der Einführhülse bilden, damit über den Formschluss eine Drehung der Applikatorspitze durch manuelles Drehen der Einführhülse erleichtert wird. Dies ist insbesondere vorteilhaft, um die Applikatorspitze ein- und auszuschrauben.

Beispielsweise kann der in Umlaufrichtung wirksame Formschluss dadurch erzielt werden, dass der radiale Vorsprung in Umfangrichtung nur segmentweise ausgeführt ist, d.h. er bildet keine umlaufend geschlossene ringförmige Anschlagfläche. Gleichzeitig kann die Einführhülse entsprechend komplementär dazu segmentweise ausgeführt sein, so dass die Einführhülse nicht nur an dem radialen Vorsprung axial anschlägt, sondern sich mit diesem formschlüssig verzahnt. Dadurch kann die Applikatorspitze über die Einführhülse von proximaler Seite aus nicht nur in axialer Richtung verschoben, sondern auch um die Längsachse gedreht werden. Ein solches Verdrehen kann das Vorschieben und Positionieren des Lichtapplikators im Gewebe erleichtern, insbesondere ein Ein- und Ausschrauben der Applikatorspitze.

Optional können die Widerstandselemente als Gewinderinge eines sich in distale Richtung verjüngenden Schraubengewindes an der Mantelfläche des Streukörperkegels ausgeführt sein. Wenn die Einführhülse mit dem radialen Vorsprung verzahnt ist, kann über ein Drehen der Einführhülse auf proximaler Seite die Applikatorspitze wie eine Schraube in das Gewebe geschraubt und diese somit im Gewebe verankert bzw. fixiert werden. Nach erfolgter Durchführung der Therapie kann dann die Applikatorspitze durch Drehen an der mit der Applikatorspitze formschlüssig verzahnten Einführhülse in entgegengesetzte Richtung aus dem Gewebe wieder entfernt werden. Sind die Widerstandselemente in der beschriebenen Weise als Schraubengewinde und in distale Richtung verjüngend ausgeführt, dann hat dies den Vorteil, dass der Applikator deutlich gewebeschonender aus dem Gewebe entfernt werden kann.

Optional kann die Einführhülse einen Innendurchmesser aufweisen, der um mindestens 10% einen Außendurchmesser des Leiterelements übersteigt, der in einem von der Einführhülse umgebenen Teil des Leiterelements liegt. Damit wird die Reibung zwischen dem von der Einführhülse umgebenen Teil des Leiterelements und der Einführhülse reduziert, sodass das Leiterelement mit verankerter Applikatorspitze bei Organbewegungen möglichst reibungsarm folgen kann.

Optional kann der Widerstand für eine axiale Bewegung des Leiterelements in der Einführhülse geringer sein als der Widerstand für eine axiale Bewegung des radialen Vorsprungs in dem Gewebe. Durch ein möglichst reibungsarmes Gleiten des Leiterelements in der Einführhülse genügt ein relativ kleiner, und damit minimalinvasiver, radialer Vorsprung am distalen Ende, um dort einen hinreichenden Widerstand zu erzeugen, durch den sich das distale Ende mit dem Organ mitbewegt. Je geringer der Widerstand für eine axiale Bewegung des Leiterelements in der Einführhülse ist, desto weniger beansprucht die Verankerung des radialen Vorsprungs das Gewebe.

Optional kann die Einführhülse mehrteilig ausgestaltet sein mit einer Innenhülse und einer die Innenhülse zumindest teilweise umgebenden Außenhülse, wobei die Innenhülse länger als die Außenhülse ist und relativ zur Außenhülse axial beweglich ist. Die mehrteilige Ausgestaltung dieser Art hat den Vorteil, dass die Außenhülse in festem Gewebe, wie etwa im Bereich der Haut, eine reibungsarme Durchführung bereitstellen kann, während die dünnere und längere Innenhülse zur Positionierung des distalen Endes des Leiterelements verwendet werden kann. Die Innenhülse kann nach der Positionierung vollständig proximalwärts aus der Außenhülse gezogen werden, damit das Leiterelement in der relativ weiten Außenhülse noch reibungsärmer gelagert ist. Die mehrteilige Ausgestaltung dieser Art hat den weiteren Vorteil, dass nach dem vollständigen proximalwärtigen Herausziehen und Entfernen der langen Innenhülse aus der kürzeren Außenhülse nur noch die kürzere starre Außenhülse dem Anwender auf proximaler Seite entgegenragt, was die weitere Handhabung wesentlich weniger stört als es eine relative weit proximal aus dem Körper ragende lange Einführhülse tun würde.

Optional kann die vorzugsweise starre Applikatorspitze am distalen Ende des Leiterelements einen hülsenförmigen proximalen Abschnitt aufweisen, der vorzugsweise einen distalen Teil eines elektrischen Leiters im Leiterelement lateral umgibt und zumindest teilweise eine radiale Wärmedämmung für die an den elektrischen Leiter abgegebene LED-Verlustwärme gegenüber der äußeren Umgebung bildet. Der lichttransparente Streukörper und der hülsenförmige proximale Abschnitt können beispielsweise im Wesentlichen einstückig aus Kunststoff, beispielsweise aus Epoxidharz, gebildet sein, wobei das Epoxidharz eine Wärmeleitfähigkeit von beispielsweise unter 2 W/(Km) bei 20°C haben kann. Der hülsenförmige proximale Abschnitt der Applikatorspitze kann wiederum ganz oder teilweise von einer oder mehreren Wärmedämmschichten umgeben sein. Der hülsenförmige proximale Abschnitt der Applikatorspitze kann zur sicheren Befestigung der Applikatorspitze am elektrischen Leiter dienen.

Optional kann der hülsenförmige proximale Abschnitt der Applikatorspitze ein distales Ende des elektrischen Leiters umgreifen und einen Formschluss mit dem elektrischen Leiter bilden. Dadurch kann eine zusätzliche Verbesserung der Befestigungssicherheit erreicht werden. Beispielsweise kann der elektrische Leiter außenseitig mindestens eine Vertiefung oder Aufweitung aufweisen, beispielweise als lokale Querschnittsverjüngung oder-vergrößerung. Dadurch kann erreicht werden, dass der hülsenförmige proximale Abschnitt der Applikatorspitze mit dem elektrischen Leiter einen Formschluss bilden kann. Die Applikatorspitze kann als Kunststoffgussteil oder als Kunststoffspritzgussteil ausgebildet sein, das beispielsweise durch Umspritzen oder Umgießen mit der außenseitigen Vertiefung oder Aufweitung des elektrischen Leiters einen Formschluss mittels eines Hinterschnitts bilden kann.

Optional kann das Volumen der Applikatorspitze im Wesentlichen vom Streukörper ausgebildet sein und in einem spitzen und/oder kantenförmigen Applikatorspitzenendbereich ein Verstärkungselement aufweisen. Dies ist besonders vorteilhaft, um eine möglichst scharfe und damit minimalinvasive lichttransparente Applikatorspitze bereitzustellen, die sowohl sicher als auch kostengünstig ist. Der Werkstoff dieses Verstärkungselements kann vorzugsweise einerseits eine hohe Festigkeit und andererseits eine hohe Zähigkeit aufweisen. Die hohe Festigkeit ist vorteilhaft für scharfe und dementsprechend dünne, aber dennoch stark belastbare Kanten und/oder Spitzen. Die hohe Zähigkeit ist vorteilhaft, um bei potenzieller Überbelastung der scharfen Kanten und/oder Spitzen ein Abbrechen von Kanten-/Spitzenteilen zu vermeiden. Dies stellt insbesondere ein Problem bei Materialien mit hoher Sprödigkeit, wie etwa Glas, dar. Ein Werkstofftyp, der beide Eigenschaften, d.h. hohe Festigkeit und hohe Zähigkeit, vereinen kann, ist beispielsweise Metall. Würde ein sprödes Glas verwendet, wäre der Applikatorspitzenendbereich bei Belastung nicht sicher gegen ein Abbrechen. Ein transparenter Kunststoff wie Epoxidharz ist nicht hart genug, also zu biegsam, um ihn im Applikatorspitzenendbereich scharf genug auszubilden, ohne dass er sich bei Belastung im Applikatorspitzenendbereich verbiegen würde. Durch das Verstärkungselement kann der Applikatorspitzenendbereich so verstärkt werden, dass ein günstig herstellbarer transparenter Kunststoff wie Epoxidharz als Basismaterial für den Streukörper der Applikatorspitze verwendet werden kann. Da ein solcher Kunststoff relativ einfach gegossen, spritzgegossen, oder in vergleichbarer anderer Form verarbeitet werden kann, ist es im Gegensatz zu Glas, Kristall, usw. auch möglich, den oben beschriebenen Formschluss zur Verbesserung der Befestigungssicherheit der Applikatorspitze am Applikator zu erzielen, indem die Applikatorspitze beispielsweise durch Umspritzen oder Umgießen der LED und eines distalen Abschnitts des elektrischen Leiters geformt wird.

Optional kann der Streukörper im Wesentlichen aus lichtstreuendem Kunststoff oder lichttransparentem Kunststoff mit lichtstreuenden Partikeln und das Verstärkungselement aus Metall, beispielsweise Stahl, ausgebildet sein. Der Anteil der Querschnittsfläche der Applikatorspitze, der vom nicht lichttransparentem Verstärkungselement eingenommen wird, kann vernachlässigbar klein sein, z. B. unter 15% liegen.

Optional kann die Mantelfläche des Streukörperkegels als ein sich in distale Richtung verjüngendes Schraubengewinde ausgeführt sein. Dadurch wird es möglich, über ein Drehen die Applikatorspitze wie eine Schraube in das Gewebe zu schrauben und diese somit dort zu verankern bzw. zu fixieren. Nach erfolgter Durchführung der Therapie kann dann die Applikatorspitze durch Drehen an der mit der Applikatorspitze formschlüssig verzahnten Einführhülse gewebeschonend aus dem Gewebe wieder entfernt werden. Wenn der Streukörperkegel als Kunststoffkegel ausgeführt wird, kann er im Gegensatz zu Glas, Kristall, usw. relativ einfach gegossen, spritzgegossen, oder in vergleichbarer anderer Form verarbeitet werden und die oben beschriebenen Schraubengewinde an der Mantelfläche des Streukörpers ausgebildet werden.

Optional kann das Verstärkungselement als ein in den Streukörper zumindest teilweise eingebetteter Dorn oder als in den Streukörper zumindest teilweise eingebettete Klinge ausgeführt sein. Die jeweils bevorzugte Ausgestaltung kann von der Anwendung abhängen. Bei einer perkutanen PDT an einem Prostatakarzinom kann es beispielsweise sein, dass der Lichtapplikator auf dem Weg zum Prostatakarzinom gesunde Nervenbahnen oder andere sensible Gefäße passiert, die nicht verletzt oder durchtrennt werden sollen. Eine dornförmige Ausführung des Verstärkungselements kann dann das Risiko einer ungewollten Verletzung von gesundem Gewebe, Nerven oder Gefäßen signifikant verringern. Bei einer perkutanen PDT an einem Mammakarzinom hingegen könnte die Verletzung oder Durchtrennung von gesundem Fettgewebe weniger problematisch sein und eine klingenförmige Ausführung des Verstärkungselements den Widerstand beim Einstechen verringern, die Narbenbildung reduzieren und den Heilungsprozess verbessern.

Optional kann das Verstärkungselement zumindest teilweise distalwärts und/oder lateral aus dem Streukörper herausragen. Dann wirkt das Verstärkungselement hauptsächlich als Einstichnadel oder -klinge und weniger der Applikatorspitzenendbereich des Streukörpers selbst, in den es zumindest teilweise eingebettet ist. Das metallische Verstärkungselement kann dann distalseitig sehr spitz und/oder scharf wie ein Skalpell ausgebildet sein.

Optional kann das Verstärkungselement zumindest teilweise den radialen Vorsprung und den axialen Anschlag für die Einführhülse bilden. Der Anschlag kann zwar umlaufend am distalen Ende angeordnet sein, muss er aber nicht. Beispielweise kann das Verstärkungselement in Form einer Klinge lateral aus dem Streukörper herausragen und so einen in Umfangrichtung gesehen nur abschnittsweise oder punktuell radialen Vorsprung mit axialem Anschlag für die Einführhülse bilden.

Optional kann der Lichtapplikator eine Arretierung aufweisen, die proximalwärtig von der Einführhülse an einer gewünschten axialen Position lösbar am Leiterelement befestigbar ist und einen axialen Anschlag für ein proximales Ende der Einführhülse bildet. Durch eine solche Arretierung wird es erleichtert, den Lichtapplikator als gesamte Einheit, beispielsweise für eine Korrektur einer bereits erfolgten Platzierung im Gewebe, zurückzuziehen, um ihn neu zu platzieren. Dabei wird es einem Anwender erspart, für ein solches Zurückziehen von der starren Einführhülse, die er zuvor noch zum Vorschieben gehalten hat, auf das flexible Leiterelement umzugreifen. Nach erfolgter Korrektur bzw. finaler Platzierung des Lichtapplikators im Gewebe kann die Arretierung gelöst werden, um die starre Einführhülse zurückziehen zu können, um dann schließlich mit der eigentlichen Therapie, der Bestrahlung des Gewebes, beginnen zu können.

Beispielsweise kann eine solche Arretierung als einfache klemmenartige Komponente ausgestaltet sein, die lösbar am flexiblen Leiterelement fixierbar ist. Befindet sich das Leiterelement in einer relativ zur Einführhülse maximal proximalen Position, d.h. wenn das distale Ende der Einführhülse am axialen Anschlag des radialen Vorsprungs anschlägt, kann durch maximal distales Platzieren der Arretierung der axiale Anschlag proximalseitig an der Einführhülse anliegend gesetzt werden, sodass die Einführhülse relativ zum Leiterelement zwischen den axialen Anschlägen fixiert ist. Die starre Einführhülse und das flexible Leiterelement mit der starren Applikatorspitze bilden also in dieser Form eine bzgl. axialer Bewegungen in jegliche Richtung feste Einheit, die anwenderfreundlich platziert werden kann.

Die Begriffe "distalwärtig" bzw. "proximalwärtig" sollen hierin eine relative Position bedeuten, die sich distal bzw. proximal von einem Anwender des Systems als Bezugsposition befindet. Die Begriffe "distalseitig" bzw. "proximalseitig" sollen hierin entsprechend Positionen an einer distalen bzw. proximalen Seite eines Gegenstands bedeuten. Die Begriffe "distalwärts" bzw. "proximalwärts" sollen hierin entsprechend Richtungen bedeuten, die sich nach distal bzw. proximal erstrecken.

Die Offenbarung ist nachfolgend anhand von in den Zeichnungen dargestellten Ausführungsbeispielen näher erläutert. Es zeigen:
Fig. 1a-c drei schematische Querschnittsdarstellungen einer ersten beispielhaften Ausführungsform eines hierin offenbarten Lichtapplikators in jeweils verschiedenen Phasen der Verwendung;
Fig. 2a-d vier schematische Querschnittsdarstellungen von verschiedenen beispielhaften Ausführungsformen einer Applikatorspitze eines hierin offenbarten Lichtapplikators;
Fig. 3 eine schematische Querschnittsdarstellung einer zweiten beispielhaften Ausführungsform eines hierin offenbarten Lichtapplikators;
Fig. 4a-c drei schematische Querschnittsdarstellungen der in Fig. 3 gezeigten beispielhaften Ausführungsform eines hierin offenbarten Lichtapplikators in jeweils verschiedenen Phasen der Verwendung; und Fig. 5a-c drei schematische Querschnittdarstellungen einer weiteren beispielhaften Ausführungsform eines hierin offenbarten Lichtapplikators in jeweils verschiedenen Phasen der Verwendung.

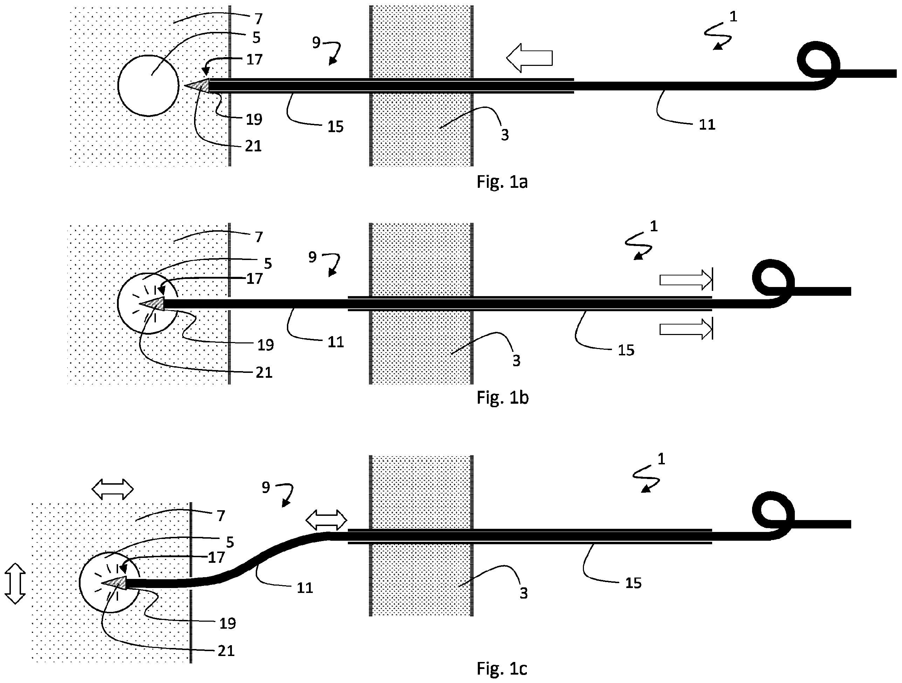

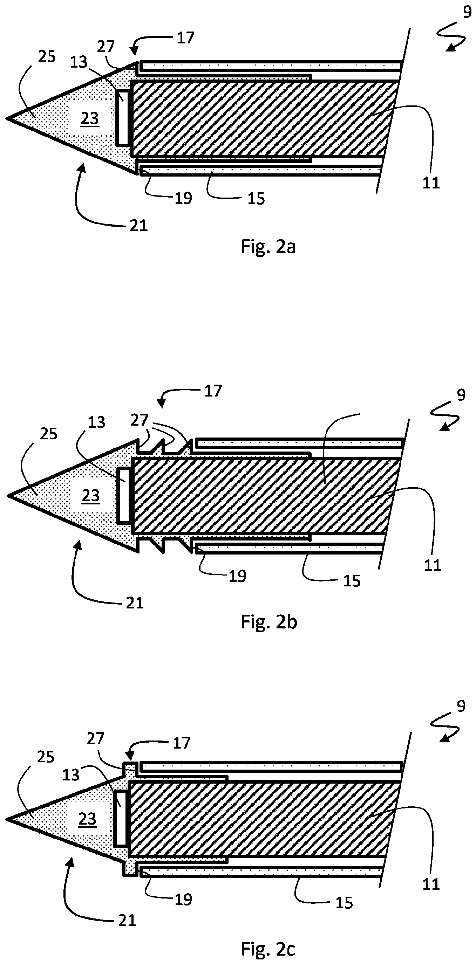

Figuren 1a-c zeigen einen Lichtapplikator 1, der perkutan durch gesundes, festes Hautgewebe 3 gestochen wurde, um malignes Gewebe 5 in einem Organ 7 mittels PDT zu behandeln. Das Organ 7 ist gegenüber dem Hautgewebe 3 beweglich, kann also beispielsweise die atmende Lunge eines Patienten sein. Der Lichtapplikator 1 weist einen distalseitigen Einführabschnitt 9 auf, der über einen proximalseitigen Anschluss (nicht gezeigt) mit einer Stromversorgungseinheit (nicht gezeigt) verbindbar ist. Der Einführabschnitt 9 weist ein flexibles Leiterelement 11 zur Stromversorgung einer LED 13 (siehe Fig. 2a-c) am distalen Ende auf. Das Leiterelement 11 ist zumindest teilweise, also zumindest entlang eines axialen Teilabschnitts des Leiterelements 11, von einer starren Einführhülse 15 umgeben. Das Leiterelement 11 ist dabei innerhalb der Einführhülse 15 relativ zur dieser axial beweglich, wobei am distalseitigen Ende des Leiterelements 11 ein radialer Vorsprung 17 mit einem axialen Anschlag 19 für die Einführhülse 15 angeordnet ist. Der radiale Vorsprung 17 ist erfindungsgemäß Is eine sich im Bereich der LED 13 angeordnete und sich distalwärts spitz verjüngende Applikatorspitze 21 mit einem lichttransparenten Streukörper zur Streuung des Lichts der LED 13 ausgebildet. Vorzugsweise ist dabei der axiale Anschlag 19 aus Metall gebildet, um ausreichend Festigkeit für die axial daran anschlagende starre Einführhülse 15 zu bieten. Es ist ganz wesentlich, dass die Applikatorspitze 21 nicht Teil der Einführhülse 15 ist, sondern am distalen Ende des Leiterelements 11 angebracht ist. Dies hat unter anderem den Vorteil, dass beim Verschieben der Einführhülse 15 gegenüber dem flexiblen Leiterelement 11 das Leiterelement 11 nicht durch die Applikatorspitze 21 beschädigt wird. Die Einführhülse 15 stößt beim Einstechen lediglich gegen den axialen Anschlag 19 und unterstützt damit die genaue Positionierung der Applikatorspitze 21 am distalen Ende des flexiblen Leiterelements 11.

In Fig. 1a ist der vorzugsweise CT- oder ultraschallunterstützte Einstechvorgang gezeigt, bei dem die Einführhülse 15 gegen den axialen Anschlag 19 stößt und die Applikatorspitze 21 einschiebt. Während des Einstechvorgangs zur Platzierung der Applikatorspitze 21 im malignen Gewebe 5 bewegt sich das Leiterelement 11 nicht relativ zur Einführhülse 15, sondern folgt ihr, da es mit der Applikatorspitze 21 verbunden ist. Die Platzierung der Applikatorspitze 21 im malignen Gewebe 5 kann mit Hilfe von Ultraschall- oder Röntgenaufnahmen für den Anwender visualisiert werden.

In Fig. 1b ist die Applikatorspitze 21 wie gewünscht im malignen Gewebe 5 positioniert. Da sich das Organ 7 allerdings bewegt und die starre Einführhülse 15 sich dabei gewebeschädigend mitbewegen würde, wird die Einführhülse 15 aus dem Organ zurückgezogen und verbleibt im Hautgewebe 3. Durch den radialen Vorsprung 17 am distalen Ende des Leiterelements 11 ist die Applikatorspitze 21 im malignen Gewebe 5 verankert und verbleibt dort. Die Einführhülse 15 wird also axial proximalwärts und relativ zum Leiterelement 11 bewegt. Dazu ist das Leiterelement 11 möglichst reibungsarm in der Einführhülse 15 gelagert, sodass es nur einer geringen Haltekraft der Applikatorspitze 21 im malignen Gewebe 5 bedarf. Um die gewünschte reibungsarme Lagerung des Leiterelements 11 innerhalb der Einführhülse 15 weiter zu verbessern, können zusätzliche, unterstützende Maßnahmen getroffen werden. Beispielsweise kann eine äußere Isolierung des Leiterelements 11 aus einem Material mit guten Gleiteigenschaften bestehen, z.B. PTFE, und/oder die äußere Isolierung kann zusätzlich mit einer biokompatiblen Gleitschicht versehen werden, z.B. mit einer Schicht aus Parylene. Das Leiterelement 11 kann in einem proximal von der Einführhülse 15 liegenden Teil unterstützend festgehalten werden, damit es nicht mit der Einführhülse 15 proximalwärts gezogen wird.

Der Verbleib der Einführhülse 15 im Hautgewebe 3 hat den Vorteil, dass sich das Leiterelement 11, wie in Figur 1c gezeigt, je nach Bewegung des Organs 7 sehr reibungsarm axial in der Einführhülse 15 bewegen kann. Ohne die Einführhülse 15 wäre die Reibung im Hautgewebe 3 höher, sodass eine größere und damit weniger gewebeschonende Applikatorspitze 21 gebraucht würde, um die Applikatorspitze 21 zu verankern. Das flexible Leiterelement 11 kann lateralen und/oder distalwärtigen und/oder proximalwärtigen Bewegungen des Organs 7 gewebeschonend folgen, sodass die Applikatorspitze 21 trotz der Bewegungen des Organs 7 während der PDT an der gewünschten Stelle im malignen Gewebe 5 verbleibt.

Nach der PDT kann das Leiterelement 11 mit der Applikatorspitze 21 proximalwärts aus dem Organ 7 gezogen werden bis der Anschlag 19 gegen die Einführhülse 15 stößt. Daraufhin kann die Einführhülse 15 zusammen mit dem Leiterelement 11 und der Applikatorspitze 21 aus dem Hautgewebe 3 gezogen werden. Alternativ dazu kann der Einführabschnitt 9 einfach ganz aus dem Körper herausgezogen werden. Eine besonders gewebeschonende Methode kann es sein, wenn die Einführhülse 15 zunächst wieder distal in den Körper eingeschoben wird bis sie an den Anschlag 19 stößt und erst dann die Einführhülse 15 zusammen mit dem Leiterelement 11 und der Applikatorspitze 21 aus dem Körper gezogen wird. Vorzugsweise kann der Applikator 1 dazu Längenmarkierungen für die relative Axialposition des Leiterelements 11 und der Einführhülse 15 zueinander aufweisen.

Figuren 2a-d zeigen das distale Ende des Einführabschnitts 9 des Lichtapplikators 1 detaillierter. Das distale Ende des Leiterelements 11 ist in elektrischem Kontakt mit der LED 13, die distalwärts durch einen lichttransparenten Streukörper 23 Licht für die PDT abstrahlt. Die Applikatorspitze 21 wird im Wesentlichen vom Streukörper 23 gebildet und verjüngt sich spitz distalwärts. Dadurch ist das Durchstechen von gesundem Gewebe auf dem Weg zum malignen Gewebe 5 sehr schonend. In einem spitzen und/oder kantenförmigen Applikatorspitzenendbereich 25 kann die Applikatorspitze 21 ein Verstärkungselement, vorzugsweise aus Metall, aufweisen. Der Streukörper 23 selbst ist im Wesentlichen aus lichtstreuendem Kunststoff oder lichttransparentem Kunststoff mit lichtstreuenden Partikeln ausgebildet. Das Verstärkungselement kann beispielsweise als in den Streukörper zumindest teilweise eingebetteter Dorn oder als in den Streukörper zumindest teilweise eingebettete Klinge ausgeführt sein. Dabei kann das Verstärkungselement zumindest teilweise distalwärts und/oder lateral aus dem Streukörper 23 herausragen. Für den Fall, dass das Verstärkungselement zumindest teilweise lateral aus dem Streukörper 23 herausragt, kann dieser laterale Überstand den radialen Vorsprung 17 und den axialen Anschlag 19 für die Einführhülse 15 bilden.

In Figuren 2a-d hat die Applikatorspitze 21 eine Pfeilform, bei der der radiale Vorsprung 17 zumindest teilweise radial über die Innenkontur der Einführhülse 15 hinausragt, aber radial innerhalb der Außenkontur der Einführhülse 15 liegt. Dadurch wird zum einen bei distalwärtigem Einschub des Einführabschnitts 9 die distale Stirnfläche der Einführhülse 15 zumindest teilweise von dem radialen Vorsprung 17 abgedeckt und zum anderen bei proximalwärtigem Rückzug des Einführabschnitts 9 der radiale Vorsprung 17 von der Einführhülse 15 abgedeckt, sodass der Widerstand für beide axialen Bewegungsrichtungen des Einführabschnitts 9 durch das Gewebe reduziert ist, was das Gewebe schont.

Der radiale Vorsprung 17 kann ein oder mehrere Widerstandselemente 27 zum Verankern des Vorsprungs 17 im Gewebe aufweisen. In Fig. 2a bildet der Anschlag 19 ein Widerstandselement 27. In Fig. 2b sind drei Widerstandselemente 27 axial hintereinander angeordnet, um die Haltekraft im malignen Gewebe 5 zu erhöhen. Der Widerstand für eine axiale Bewegung des Leiterelements 11 in der Einführhülse 15 kann dann vorzugsweise geringer sein als der Widerstand für eine axiale Bewegung des radialen Vorsprungs 17 bzw. der Widerstandselemente 27 in dem malignen Gewebe 5. Fig. 2c zeigt eine weitere Ausführungsform mit einem vom Anschlag 19 gebildeten Widerstandselement 27, das weniger gewebeschonend für die distalwärtige Einstichbewegung ist.

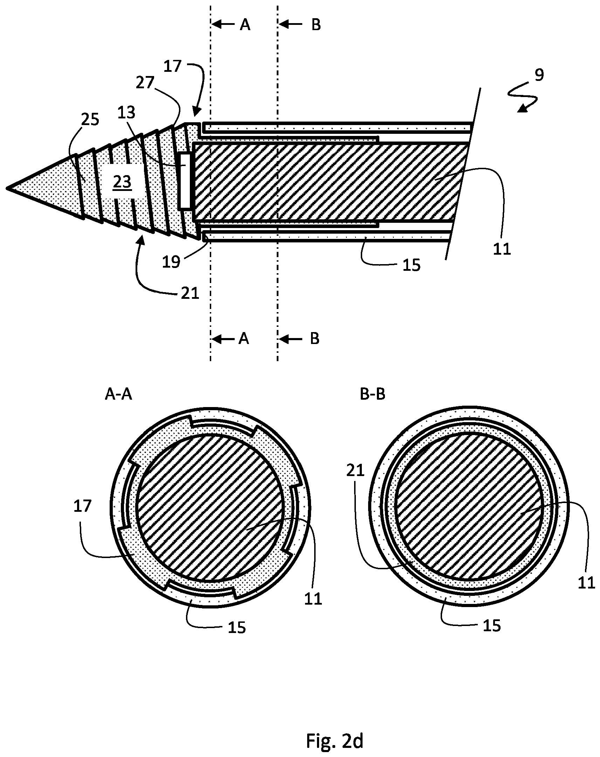

Fig. 2d zeigt eine Ausführungsform, bei welcher mehrere Widerstandselemente 27 in Form von Gewindestegen eines sich distalwärts verjüngenden Schraubengewindes ausgeführt sind. Dabei ist der eigentliche Streukörper 23 und die Helix des Schraubengewindes einstückig aus lichtstreuendem Kunststoff oder lichttransparentem Kunststoff mit lichtstreuenden Partikeln ausgebildet, so dass die gewünschte isotrope Bestrahlung des Gewebes 5, welche in den Fig. 2a bis 2c mit einer glatten Kegelmantelfläche erreicht werden konnte, auch mit dem in den Streukörper integrierten Schraubengewinde aufrecht erhalten werden kann. Außerdem ist, wie in der Querschnittsansicht A-A gezeigt, der axiale Anschlag 19 der Applikatorspitze 21 nicht durch einen kontinuierlich umlaufenden Vorsprung 17, sondern durch einen in Umlaufrichtung segmentweisen Vorsprung 17 gebildet. Entsprechend ist das distale Ende der Einführhülse 15 segmentweise komplementär zum Vorsprung 17 ausgebildet. Dadurch wird bei entsprechender Drehstellung der Einführhülse 15 gegenüber der Applikatorspitze 21 eine formschlüssige Verzahnung von Applikatorspitze 21 und Einführhülse 15 möglich, die wiederum erlaubt, dass die Applikatorspitze 21 über ein proximalseitiges Drehen der Einführhülse 15 in das Gewebe bzw. den Tumor hineingeschraubt und somit in diesem verankert bzw. fixiert werden kann. Nach erfolgter Durchführung der Therapie kann dann die Applikatorspitze 21 und der gesamte Lichtapplikator 1 einfach aus dem Gewebe bzw. dem Körper herausgezogen werden. Da sich die Widerstandselemente 27 gegenüber der Ausführung der Fig. 2b nach distal verjüngen, ist dieser Vorgang vergleichsweise gewebeschonend. Soll der Vorgang noch gewebeschonender erfolgen, dann kann die Einführhülse 15 noch einmal durch entsprechende Drehstellung mit der Applikatorspitze verzahnt werden und durch Drehen der Einführhülse 15 auf proximaler Seite in die entgegengesetzte Richtung die Applikatorspitze 21 aus dem Gewebe bzw. dem Tumor herausgeschraubt werden. Der durch den Vorsprung 17 gebildete axiale Anschlag 19 kann durch den Streukörper 23 selbst und/oder durch eine vorzugsweise metallische Stabilisierungshülse gebildet sein. Eine solche Stabilisierungshülse kann ein fester Teil der Applikatorspitze 21 sein und zumindest teilweise den Teil der Applikatorspitze 21 umgeben oder bilden, der in die Einführhülse 15 hineinragt, wenn die Einführhülse 15 am axialen Anschlag 19 anschlägt.

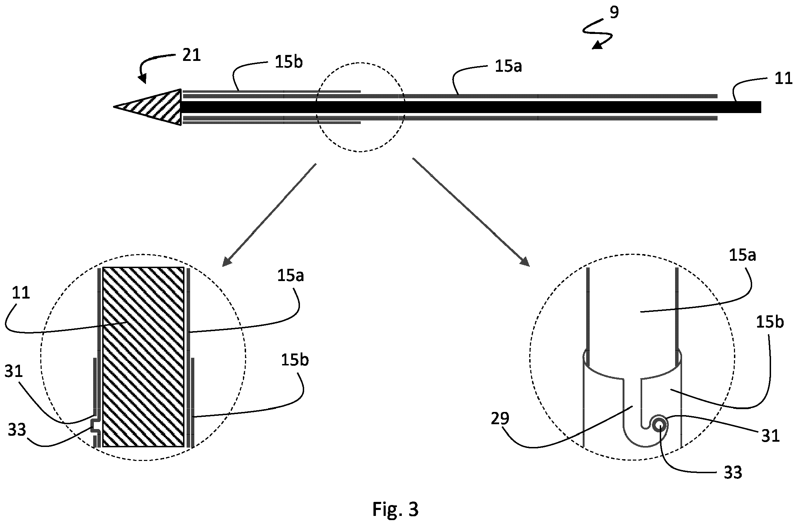

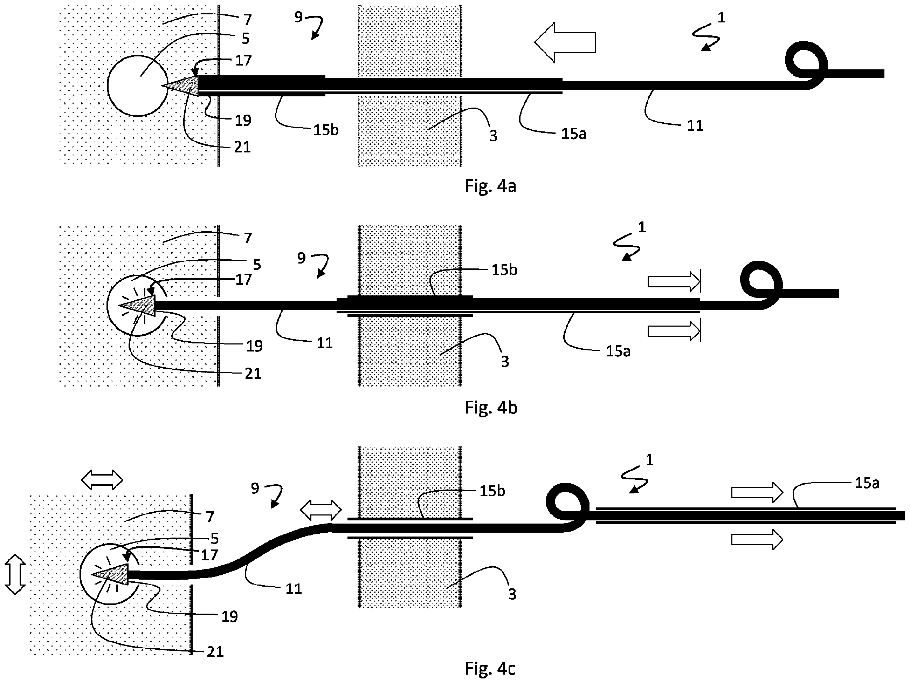

Figur 3 und Figuren 4a-c zeigen ein Ausführungsbeispiel mit mehrteiliger Einführhülse 15. Die Einführhülse 15 weist dabei eine Innenhülse 15a und eine die Innenhülse 15a zumindest teilweise, also zumindest entlang eines axialen Teilabschnitts der Innenhülse 15a, umgebende Außenhülse 15b auf. Die Innenhülse 15a ist dabei länger als die Außenhülse 15b und relativ zur Außenhülse 15b axial beweglich. In der gezeigten Ausführungsform stoßen die Innenhülse 15a und/oder die Außenhülse 15b bei einer maximal distalwärtigen Axialposition relativ zum flexiblen Leiterelement 11 an den Anschlag 19 der Applikatorspitze 21. Dies ist die in Fig. 4a gezeigte Position während des Einstechens des Einführabschnitts 9. Wie in den Detailansichten von Figur 3 zu sehen, ist die Außenhülse 15b über eine bajonettverschlussartige lösbare Kupplung mit der Innenhülse 15a koppelbar, um für bestimmte Bewegungen eine axiale Relativbewegung zwischen der Innenhülse 15a und der Außenhülse 15b zu vermeiden und für andere Bewegungen eine axiale Relativbewegung zwischen der Innenhülse 15a und der Außenhülse 15b zuzulassen. Dazu weist die Außenhülse 15b hier an ihrem proximalen Ende eine hakenförmige Kulissenführung in Form eines Schlitzes 29 auf. Der Schlitz 29 ist zum proximalen Ende der Außenhülse 15b hin offen und bildet einen Hinterschnitt 31. Die Innenhülse 15a weist eine radiale Nase 33 auf, die für die Kupplung der Innenhülse 15a und Außenhülse 15b aneinander in dem Schlitz 29 geführt ist. Die Innenhülse 15a kann dadurch nicht proximalwärts aus der Außenhülse 15b gezogen werden, wenn sich die Nase 33 wie gezeigt im Hinterschnitt 31 des Schlitzes 29 befindet. Durch die im Schlitz 29 eingehakte Nase 33 zieht die Innenhülse 15a die Außenhülse 15b dabei proximalwärts mit. Falls dies nicht gewünscht ist, kann durch Drehung der Innenhülse 15a gegenüber der Außenhülse 15b die Nase 33 über den Schlitz 29 proximalwärts aus der Außenhülse 15b geführt werden, sodass Innenhülse 15a und Außenhülse 15b voneinander gelöst sind. Dann ist es möglich, die Innenhülse 15a aus der Außenhülse 15b zu ziehen. Soll die Kupplung von Innenhülse 15a und Außenhülse 15b noch besser bzw. sicherer funktionieren, dann kann der vorausgehend beschriebene bajonettartige Verschluss auch in mehrfacher Form ausgeführt werden.

Die Figuren 4a-c zeigen dieses Ausführungsbeispiel mit mehrteiliger Einführhülse 15 in jeweils verschiedenen Phasen der Verwendung analog zu Figuren 1a-c. In Figur 4a wird der Einführabschnitt 9 durch das Hautgewebe 3 in das maligne Gewebe 5 in dem Organ 7 gestochen. Die Außenhülse 15b, die in diesem Fall kürzer ist als die Eindringtiefe der Applikatorspitze 21, wird durch die radiale Nase 33 (siehe Figur 3) distalwärts von der längeren Innenhülse 15a mitgeführt. Sobald die Applikatorspitze 21 wie gewünscht im malignen Gewebe 5 positioniert ist, können die miteinander gekoppelten Hülsen 15a, 15b proximalwärts aus dem sich bewegenden Organ 7 gezogen werden (siehe Fig. 4b). Die Außenhülse 15b soll allerdings im Hautgewebe 3 verbleiben, um einen reibungsarmen Kanal für das Leiterelement 11 durch das Hautgewebe 3 zu schaffen. Wenn lediglich die kürzere Außenhülse 15b im Hautgewebe 3 verbleibt, hat dies den weiteren Vorteil, dass sie, wenn sie die Position von Fig. 4b und 4c erreicht hat, erstens proximalwärtig weniger weit in den Raum außerhalb des Patienten ragt (vgl. mit Fig. 1b und 1c) und damit die weitere Handhabung weniger stört und zweitens durch eine durch Eigengewicht bedingte geringere Hebelwirkung auf die Einstichstelle an der Haut 3 gewebeschonender ist. Weiter kommt in vorteilhafter Weise hinzu, dass bei der kürzeren Außenhülse 15b auch die Berührungsfläche mit dem Leiterelement 11 kleiner ist als bei der längeren Innenhülse 15a, was die Reibungskräfte zwischen Leiterelement 11 und Einführhülse 15 in gewünschter Weise weiter reduziert. Sobald die Außenhülse 15b wie gewünscht im Hautgewebe 3 positioniert ist, kann die Innenhülse 15a von der Außenhülse 15b gelöst werden und relativ zu dieser proximalwärts vollständig aus dem Körper gezogen werden, wobei das Leiterelement 11 durch die Verankerung der Applikatorspitze 21 im malignen Gewebe 5 nicht mit proximalwärts herausgezogen wird. Das Leiterelement 11 kann dabei in einem proximal von der Innenhülse 15a liegenden Teil unterstützend festgehalten werden, damit es nicht mit der Innenhülse 15a proximalwärts gezogen wird. Sobald die Innenhülse 15a komplett aus der Außenhülse 15b gezogen ist (siehe Figur 4c), hat das Leiterelement 11 in der Außenhülse 15b relativ großes radiales Spiel und dadurch eine besonders reibungsarme Lagerung in der Außenhülse 15b. Somit kann es den Bewegungen des Organs 7 folgen, sodass die Applikatorspitze 21 während der Dauer der PDT an der gewünschten Stelle im malignen Gewebe 5 verbleibt und dabei gleichzeitig das gesunde durchstochene Gewebe geschont wird.

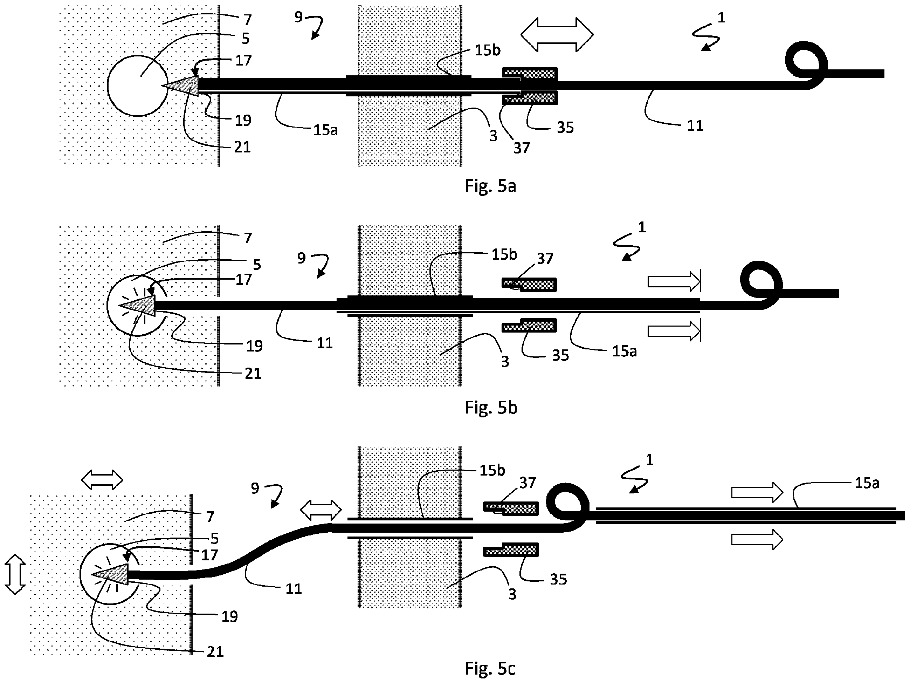

Das in Figuren Fig. 5a-c gezeigte Ausführungsbeispiel weist im Gegensatz zu dem in Figuren 4a-c gezeigten Ausführungsbeispiel eine Arretierung 35 in Form einer klemmenartigen Komponente auf, die lösbar am flexiblen Leiterelement 11 fixierbar ist und einen proximalen axialen Anschlag 37 für die Einführhülse 15a, 15b aufweist. Die Arretierung 35 kann bei einer einteiligen Einführhülse 15 oder mehrteiligen Einführhülse 15a, 15b eingesetzt werden. In Fig. 5a befindet sich das Leiterelement 11 in einer relativ zur Innenhülse 15a maximal proximalen Position, d.h. das distale Ende der Innenhülse 15a schlägt am axialen Anschlag 19 des radialen Vorsprungs 17 an. Die proximal von der Innenhülse 15a angeordnete Arretierung 35 hat eine maximal distalwärtige Position, sodass die Innenhülse 15a relativ zum Leiterelement 11 zwischen dem Anschlag 19 und dem Anschlag 37 fixiert ist. Die Innenhülse 15a und das flexible Leiterelement 11 mit der starren Applikatorspitze 21 bilden also in dieser Form eine bzgl. axialer Bewegungen in jegliche Richtung feste Einheit, die anwenderfreundlich und gewebeschonend platziert werden kann. Bis zur endgültigen Platzierung der Einheit kann diese relativ reibungsarm durch die Außenhülse 15b vor- und zurückbewegt werden, um die endgültige Platzierung für die Behandlung zu finden.

In Figuren 5b und 5c ist die endgültige Platzierung für die Behandlung gefunden, sodass die Arretierung 35 gelöst werden kann und die Innenhülse 15a proximalwärts vollständig aus der Außenhülse 15b gezogen werden kann, damit die Applikatorspitze 21 während der Behandlung den Bewegungen des Organs 7 leicht und gewebeschonend folgen kann. Die anhand der Fig. 5a-c dargestellte Vorgehensweise hat gegenüber der anhand der Fig. 4a-c dargestellten Vorgehensweise u.a. den Vorteil, dass die Außenhülse 15b nicht zuerst distalwärts bis zum Tumor 5 bewegt wird, um anschließend wieder proximalwärts in den Bereich der Haut 3 zurückbewegt zu werden. Das bedeutet eine Reduzierung der Gewebebelastung und eine Reduzierung der Behandlungszeit. Wenn außerdem lediglich die Innenhülse 15a und nicht auch noch die Außenhülse 15b am Anschlag 19 anschlagen oder zumindest von diesem in radiale Richtung abgedeckt werden muss, dann kann des Weiteren der radiale Vorsprung 17 mit einer geringeren lateralen Ausdehnung ausgeführt werden, was eine weitere Reduzierung der Gewebebelastung bedeutet. Dies ist ein weiterer Vorteil der Vorgehensweise von Fig. 5a-c gegenüber der Vorgehensweise von Fig. 4a-c.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben. Entsprechend soll der Schutzbereich der Ansprüche solche äquivalente Ausführungsformen umfassen. Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Jedes Merkmal, das im Rahmen einer Ausführungsform offenbart wurde, kann allein oder in Kombination mit einem oder mehreren anderen Merkmalen verwendet werden, unabhängig davon, in welcher Ausführungsform die Merkmale jeweils beschrieben wurden. Während mindestens ein Ausführungsbeispiel hierin beschrieben und gezeigt ist, seien Abwandlungen und alternative Ausführungsformen, die einer fachmännisch versierten Person in Anbetracht dieser Beschreibung als offensichtlich erscheinen, vom Schutzbereich dieser Offenbarung mit erfasst. Im Übrigen soll hierin weder der Begriff "aufweisen" zusätzliche andere Merkmale oder Verfahrensschritte ausschließen noch soll "ein" oder "eine" eine Mehrzahl ausschließen.

### Bezugszeichenliste:

- 1: Lichtapplikator
- 3: Hautgewebe
- 5: malignes Gewebe
- 7: Organ
- 9: Einführabschnitt
- 11: Leiterelement
- 13: LED
- 15: Einführhülse
- 15a: Innenhülse
- 15b: Außenhülse
- 17: radialer Vorsprung
- 19: Anschlag
- 21: Applikatorspitze
- 23: Streu körper
- 25: Applikatorspitzenendbereich
- 27: Widerstandselement
- 29: Schlitz
- 31: Hinterschnitt
- 33: Nase
- 35: Arretierung
- 37: Anschlag

## Patentansprüche

1. Lichtapplikator (1) zur Untersuchung und/oder Behandlung von einem organischen Körper, wobei der Lichtapplikator einen distalseitigen Einführabschnitt (9) mit einer LED (13) am distalen Ende zum Einstechen in Gewebe (5) des organischen Körpers aufweist, wobei der Einführabschnitt (9) ein flexibles Leiterelement (11) zur Stromversorgung der LED (13) und eine das Leiterelement (11) zumindest teilweise umgebende starre Einführhülse (15) aufweist, wobei das Leiterelement (11) relativ zur starren Einführhülse (15) axial beweglich ist, wobei am distalseitigen Ende des Leiterelements (11) ein radialer Vorsprung (17) mit einem axialen Anschlag (19) für ein distales Ende der Einführhülse (15) angeordnet ist, wobei der radiale Vorsprung (17) als eine sich distalwärts spitz verjüngende Applikatorspitze (21) mit einem lichttransparenten Streukörper (23) zur Streuung des Lichts der LED (13) ausgebildet ist.

2. Lichtapplikator (1) nach Anspruch 1, wobei der radiale Vorsprung (17) zumindest teilweise radial über die Innenkontur der Einführhülse (15) hinausragt, aber radial innerhalb der Außenkontur der Einführhülse (15) liegt.

3. Lichtapplikator (1) nach einem der vorhergehenden Ansprüche, wobei der radiale Vorsprung (17) ein oder mehrere Widerstandselemente (27) zum Verankern des Vorsprungs (17) im Gewebe (5) aufweist.

4. Lichtapplikator (1) nach Anspruch 3, wobei die Widerstandselemente (27) als sich vorzugsweise verjüngende Gewindestege ausgebildet sind.

5. Lichtapplikator (1) nach einem der vorhergehenden Ansprüche, wobei der radiale Vorsprung (17) einen in Umlaufrichtung wirksamen Formschluss mit dem distalen Ende der Einführhülse (15) bildet.

6. Lichtapplikator (1) nach einem der vorhergehenden Ansprüche, wobei die Einführhülse (15) einen Innendurchmesser aufweist, der um mindestens 10% einen Außendurchmesser des Leiterelements (11) übersteigt, der in einem von der Einführhülse (15) umgebenen Teil des Leiterelements (11) liegt.

7. Lichtapplikator (1) nach einem der vorhergehenden Ansprüche, wobei der Widerstand für eine axiale Bewegung des Leiterelements (11) in der Einführhülse (15) geringer ist als der Widerstand für eine axiale Bewegung des radialen Vorsprungs (17) in dem Gewebe (5).

8. Lichtapplikator (1) nach einem der vorhergehenden Ansprüche, wobei die Einführhülse (15) mehrteilig ausgestaltet ist mit einer Innenhülse (15a) und einer die Innenhülse (15a) zumindest teilweise umgebenden Außenhülse (15b), wobei die Innenhülse (15a) länger als die Außenhülse (15b) ist und relativ zur Außenhülse (15b) axial beweglich ist.

9. Lichtapplikator (1) nach Anspruch 8, wobei die Einführhülse (15) eine lösbare Kupplung zwischen Innenhülse (15a) und Außenhülse (15b) aufweist, um wahlweise für bestimmte Bewegungen eine axiale Relativbewegung zwischen der Innenhülse (15a) und der Außenhülse (15b) zu vermeiden und für andere Bewegungen eine axiale Relativbewegung zwischen der Innenhülse (15a) und der Außenhülse (15b) zuzulassen.

10. Lichtapplikator (1) nach einem der vorhergehenden Ansprüche, wobei das Volumen der Applikatorspitze (21) im Wesentlichen vom Streukörper (23) ausgebildet wird und in einem spitzen und/oder kantenförmigen Applikatorspitzenendbereich (25) ein Verstärkungselement aufweist.

11. Lichtapplikator (1) nach Anspruch 10, wobei der Streukörper (23) im Wesentlichen aus lichtstreuendem Kunststoff oder lichttransparentem Kunststoff mit lichtstreuenden Partikeln ausgebildet ist und das Verstärkungselement aus Metall ist.

12. Lichtapplikator (1) nach Anspruch 10 oder 11, wobei das Verstärkungselement als in den Streukörper (23) zumindest teilweise eingebetteter Dorn oder als in den Streukörper (23) zumindest teilweise eingebettete Klinge ausgeführt ist.

13. Lichtapplikator (1) nach einem der Ansprüche 10 bis 12, wobei das Verstärkungselement zumindest teilweise distalwärts und/oder lateral aus dem Streukörper (23) herausragt.

14. Lichtapplikator (1) nach einem der Ansprüche 10 bis 13, wobei das Verstärkungselement zumindest teilweise den radialen Vorsprung (17) und den axialen Anschlag (19) für die Einführhülse (15) bildet.

15. Lichtapplikator (1) nach einem der Ansprüche 11 bis 14, wobei die sich distalwärts spitz verjüngende Applikatorspitze (21) Gewindestege als Widerstandselemente (27) zum Verankern der Applikatorspitze (21) im Gewebe (5) aufweist.

## Claims

1. Light applicator (1) for examining and/or treating an organic body, wherein the light applicator comprises an insertion section (9) on the distal side with an LED (13) at the distal end for piercing into tissue (5) of the organic body,
wherein the insertion section (9) comprises a flexible conductor element (11) for supplying the LED (13) with power and a rigid insertion sleeve (15) at least partially surrounding the conductor element (11), wherein the conductor element (11) is axially moveable relative to the rigid insertion sleeve (15), wherein a radial projection (17) with an axial stop (19) for a distal end of the insertion sleeve (15) is arranged on the distal end of the conductor element (11), wherein the radial projection (17) is designed as an applicator tip (21) pointedly tapering in the distal direction with a light-transparent scatter body (23) for scattering the light of the LED (13).

2. Light applicator (1) according to claim 1, wherein the radial projection (17) at least partially projects radially beyond the inner contour of the insertion sleeve (15) but lies radially within the outer contour of the insertion sleeve (15).

3. Light applicator (1) according to any one of the preceding claims, wherein the radial projection (17) comprises one or more resistance elements (27) for anchoring the projection (17) in the tissue (5).

4. Light applicator (1) according to claim 3, wherein the resistance elements (27) are designed as preferably tapering threaded steps.

5. Light applicator (1) according to any one of the preceding claims, wherein the radial projection (17) forms a positive locking fit with the distal end of the insertion sleeve (15) that acts in the circumferential direction.

6. Light applicator (1) according to any one of the preceding claims, wherein the insertion sleeve (15) has an inner diameter that exceeds by at least 10% an outer diameter of the conductor element (11) that lies in a part of the conductor element (11) surrounded by the insertion sleeve (15).

7. Light applicator (1) according to any one of the preceding claims, wherein the resistance for an axial movement of the conductor element (11) in the insertion sleeve (15) is less than the resistance for an axial movement of the radial projection (17) in the tissue (5).

8. Light applicator (1) according to any one of the preceding claims, wherein the insertion sleeve (15) is designed in multiple parts with an inner sleeve (15a) and an outer sleeve (15b) at least partially surrounding the inner sleeve (15a), wherein the inner sleeve (15a) is longer than the outer sleeve (15b) and axially moveable relative to the outer sleeve (15b).

9. Light applicator (1) according to claim 8, wherein the insertion sleeve (15) has a detachable coupling between the inner sleeve (15a) and the outer sleeve (15b) to optionally prevent an axial relative movement between the inner sleeve (15a) and the outer sleeve (15b) for certain movements, and for other movements to allow an axial relative movement between the inner sleeve (15a) and the outer sleeve (15b).

10. Light applicator (1) according to any one of the preceding claims, wherein the volume of the applicator tip (21) is essentially formed by the scatter body (23) and comprises a reinforcing element in a pointed and/or edge-shaped applicator tip end area (25).

11. Light applicator (1) according to claim 10, wherein the scatter body (23) is essentially made of light-scattering plastic or light-transparent plastic with light-scattering particles and the reinforcing element is made of metal.

12. Light applicator (1) according to claim 10 or 11, wherein the reinforcing element is designed as spike at least partially embedded in the scatter body (23) or as blade at least partially embedded in the scatter body (23).

13. Light applicator (1) according to any one of claims 10 to 12, wherein the reinforcing element projects at least partially in the distal and/or lateral direction from the scatter body (23).

14. Light applicator (1) according to any one of claims 10 to 13, wherein the reinforcing element at least partially forms the radial projection (17) and the axial stop (19) for the insertion sleeve (15).

15. Light applicator (1) according to any one of claims 11 to 14 wherein the distally pointedly tapering applicator tip (21) comprises threaded steps as resistance elements (27) for anchoring the applicator tip (21) in the tissue (5).

## Revendications

1. Applicateur de lumière (1) pour examiner et/ou traiter un corps organique, dans lequel l'applicateur lumineux présente un tronçon d'introduction (9) distal avec une LED (13) à l'extrémité distale pour piquer dans le tissu (5) du corps organique,
dans lequel le tronçon d'introduction (9) présente un élément conducteur flexible (11) pour l'alimentation en courant électrique de la LED (13) et un manchon d'introduction fixe (15) entourant au moins partiellement l'élément conducteur flexible (11), dans lequel l'élément conducteur flexible (11) est mobile axialement par rapport au manchon d'introduction fixe (15), dans lequel une saillie radiale (17) avec une butée axiale (19) pour une extrémité distale du manchon d'introduction (15) est disposée sur l'extrémité distale de l'élément conducteur (11), dans lequel la saillie radiale (17) est conçue en tant qu'une pointe d'applicateur (21) se rétrécissant en pointe en direction distale, avec un corps de diffusion transparent à la lumière (23) pour diffuser la lumière de la LED (13).

2. Applicateur de lumière (1) selon la revendication 1, dans lequel la saillie radiale (17) fait saillie radialement au moins partiellement sur le contour intérieur du manchon d'introduction (15), mais repose radialement à l'intérieur du contour extérieur du manchon d'introduction (15).

3. Applicateur de lumière (1) selon l'une des revendications précédentes, dans lequel la saillie radiale (17) présente un ou plusieurs éléments de résistance (27) pour l'ancrage de la saillie (17) dans le tissu (5).

4. Applicateur de lumière (1) selon la revendication 3, dans lequel les éléments de résistance (27) sont conçus en tant que tiges filetées se rétrécissant de préférence.

5. Applicateur de lumière (1) selon l'une des revendications précédentes, dans lequel la saillie radiale (17) forme un accouplement par adhérence de formes agissant dans le sens périphérique avec l'extrémité distale du manchon d'introduction (15).

6. Applicateur de lumière (1) selon l'une des revendications précédentes, dans lequel le manchon d'introduction (15) présente un diamètre intérieur qui excède d'au moins 10 % un diamètre extérieur de l'élément conducteur (11), qui repose dans une partie de l'élément conducteur (11) entourée par le manchon d'introduction (15).

7. Applicateur de lumière (1) selon l'une des revendications précédentes, dans lequel la résistance pour un mouvement axial de l'élément conducteur (11) dans le manchon d'introduction (15) est plus faible que la résistance pour un mouvement axial de la saillie radiale (17) dans le tissu (5).

8. Applicateur de lumière (1) selon l'une des revendications précédentes, dans lequel le manchon d'introduction (15) est conçu en plusieurs parties avec un manchon intérieur (15a) et un manchon extérieur (15b) entourant le manchon intérieur (15a) au moins partiellement, dans lequel le manchon intérieur (15a) est plus long que le manchon extérieur (15b) et est mobile axialement par rapport au manchon extérieur (15b).

9. Applicateur de lumière (1) selon la revendication 8, dans lequel le manchon d'introduction (15) présente un couplage séparable entre le manchon intérieur (15a) et le manchon extérieur (15b), pour au choix éviter un mouvement relatif axial entre le manchon intérieur (15a) et le manchon extérieur (15b) pour certains mouvements et autoriser un mouvement relatif axial entre le manchon intérieur (15a) et le manchon extérieur (15b) pour d'autres mouvements.

10. Applicateur de lumière (1) selon l'une des revendications précédentes, dans lequel le volume de la pointe d'applicateur (21) est conçu essentiellement par le corps de diffusion (23) et présente un élément de renforcement dans une zone d'extrémité de pointe d'applicateur (25) pointue et/ou en arête.

11. Applicateur de lumière (1) selon la revendication 10, dans lequel le corps de diffusion (23) est conçu essentiellement de plastique diffusant la lumière ou de plastique transparent à la lumière avec des particules diffusant la lumière, et l'élément de renforcement est en métal.

12. Applicateur de lumière (1) selon la revendication 10 ou 11, dans lequel l'élément de renforcement est conçu en tant que poinçon enchâssé au moins partiellement dans le corps de diffusion (23) ou en tant que lame enchâssée au moins partiellement dans le corps de diffusion (23).

13. Applicateur de lumière (1) selon l'une des revendications 10 à 12, dans lequel l'élément de renforcement dépasse au moins partiellement du corps de diffusion (23) dans la direction axiale et/ou latéralement.

14. Applicateur de lumière (1) selon l'une des revendications 10 à 13, dans lequel l'élément de renforcement forme au moins partiellement la saillie radiale (17) et la butée axiale (19) pour le manchon d'introduction (15).

15. Applicateur de lumière (1) selon l'une des revendications 11 à 14, dans lequel la pointe d'applicateur (21) se rétrécissant en pointe en direction distale présente des tiges filetées en tant qu'éléments de résistance (27) pour ancrer la pointe d'applicateur (21) dans le tissu (5).
